# EUROPEAN PATENT APPLICATION

(11) **EP 2 045 008 A2**
(43) Date of publication of application: **08.04.2009**
(21) Application number: 08163679.7
(22) Date of filing: 04.09.2008
(51) Int. Cl.: B01J 23/52, B01J 37/02, C07C 67/055

(54) **Catalyst**

(30) Priority: 04.10.2007 DE 102007047430
(71) Applicant: Evonik Degussa GmbH, 45128 Essen (DE)
(72) Inventor: Mayer, Ralf, 45130 Essen (DE); Schimmer, Klaus, 69221 Dossenheim (DE); Renneke, Roman, Paducah, KY 42003 (US); Arunajatesan, Venugopal, Paducah, KY 42003 (US); Geisselmann, Andreas, 63075 Offenbach am Main (DE); Lansink Rotgerink, Hermanus Gerhardus Jozef, 63776 Mömbris/Mensengesäß (DE)

(57) **Abstract**

Catalyst comprising palladium, gold and alkali metal acetate as catalytically active components on a support, which is modified by means of titanium, iron, lanthanum, cerium, yttrium and/or molybdenum or oxides thereof.

It can be used for preparing vinyl acetate monomer.

## Description

The invention relates to a catalyst, a process for producing it and its use for preparing vinyl acetate monomers.

Supported catalysts containing gold, palladium and alkali metal compounds are used for the production of vinyl acetate. For this purpose, ethene, acetic acid and molecular oxygen or air are reacted in the gas phase, with or without addition of inert gases, at temperatures in the range from 100 to 250°C and under ambient or superatmospheric pressure in the presence of the supported catalyst.

Processes for preparing vinyl acetate monomer are known from DE 16 68 088, EP-A 0 464 633, EP-A-0 519 435,

EP-A 0 634 208, EP-A 0 723 810, EP-A 0 634 209,

EP-A 0 632 214, EP-A 0 654 301, EP-A 0 723 810,

US 4,048,096, US 5,185,308 and US 5,371,277. These documents also describe processes for producing supported catalysts. Depending on the embodiment, supported catalysts having a homogeneous distribution of noble metal over the cross section of the support and catalyst having a more or less pronounced shell profile are produced.

DE-B 21 00 778, US 4,902,823, US 5,250,487, US 5,292,931, US 5,808,136, EP 0 807 615, EP 0 916 402, EP 0 997 192 A1,

EP 1 323 469 A2 and EP 0 987 058 disclose the use of shaped bodies based on pyrogenic silicon dioxides as catalyst supports in the preparation of vinyl acetate monomer.

EP-A 0 464 633 describes a supported catalyst for preparing vinyl acetate monomer, which is based on a catalyst support having at least one channel through which fluid can pass. In particular, reference is made to a hollow cylinder in which at least 95% of the palladium, gold and/or compounds thereof is located in a region from the surface to 0.5 mm below the surface of the support.

These patent texts also disclose a process for producing the supported catalysts containing gold, palladium and alkali metal compounds. Depending on the embodiment, catalysts having a homogeneous noble metal distribution over the cross section of the support and catalysts having a more or less pronounced shell profile are obtained.

These catalysts are usually obtained by impregnating the support with a basic solution and a solution containing gold and palladium salts, with the impregnations occurring simultaneously or in succession, with or without intermediate drying. The support is subsequently washed to remove any chloride present. Before or after washing, the insoluble noble metal compounds precipitated on the support are reduced. The catalyst precursor obtained in this way is dried and impregnated with alkali metal acetates or alkali metal compounds which are converted completely or partly into alkali metal acetates under the reaction conditions in the production of vinyl acetate monomer in order to activate the catalyst. Preferred alkali metal compounds are potassium compounds, in particular potassium acetate.

The reduction of the catalyst can be carried out in the aqueous phase or in the gas phase. To carry out the reduction in the aqueous phase, suitable reducing agents are, for example, formaldehyde or hydrazine. The reduction in the gas phase can be effected using hydrogen or a hydrogen/nitrogen mixture (95% by volume of N₂ + 5% by weight of H₂) or ethene. According to EP 0 634 209, the reduction is carried out using hydrogen at temperatures in the range from 40 to 260°C, preferably from 70 to 200°C. However, the catalyst is frequently reduced by means of ethene directly in the production reactor only after activation by means of alkali metal acetate.

In the production process, the catalyst is firstly supplied only slowly with the reactants. During this start-up phase, the activity of the catalyst increases and usually reaches its final level only after days or weeks.

The document EP 0 431 478 describes a process for preparing vinyl acetate in the gas phase from ethylene, acetic acid and oxygen over a catalyst containing palladium and gold on a support. The support comprises an SiO₂/Al₂O₃ mixture, with the support particles being pressed with the aid of Li, Mg, Al, Zn or Mn salts of a C₂-C₂₀-carboxylic acid as binder.

The document EP 0 723 810 A1 describes a supported catalyst for the production of vinyl acetate monomer, which contains palladium, gold, and alkali metal acetate as catalytically active components on a support composed of silicon dioxide, aluminosilicate or aluminium oxide, with the support additionally containing at least one element of groups I A, II A, III A and IV B of the Periodic Table.

The invention provides a catalyst comprising palladium, gold and alkali metal acetate as catalytically active components on a support, which is characterized in that the catalyst is modified by means of titanium, iron, lanthanum, cerium, yttrium and/or molybdenum or oxides thereof.

The support material can advantageously contain from 0.1 to 10% by weight of the respective doping components.

As support material, it is possible to use aluminosilicate, for example KA-160 from Südchemie, silicon dioxide, for example Aerolyst 3045 (Degussa), or titanium dioxide, for example Aerolyst 7751 (Degussa).

The invention further provides a process for producing the catalyst, which is characterized in that the support is impregnated with a salt of at least one doping component, calcined and subsequently impregnated with the catalytically active component or the support material is mixed with an oxide of the doping component, shaped and subsequently impregnated with the catalytically active components.

In one embodiment of the invention, at least one doping component can be applied to the support simultaneously with the catalytically active component.

The catalyst of the invention has the advantage that the proportion of noble metal in the catalyst required to achieve the known space-time yield can be reduced.

### Examples

The support material of example catalyst A comprises 4% of yttrium oxide and 96% of silicon dioxide (Degussa Aerolyst 3045). Active components are palladium, gold and potassium acetate.

Comparative catalyst B comprises only the active components palladium, gold and potassium acetate applied to an aluminosilicate as support material (Südchemie KA-160).

In the case of example catalyst C, the active components palladium, gold and potassium acetate were applied to a support material comprising 4.5% of titanium dioxide, 4.5% of iron oxide and 91% of silicon dioxide (Degussa Aerolyst 3045).

Example catalyst D comprises the active components applied to a titanium dioxide and modified with 0.3% of cerium oxide, 0.8% of iron oxide and 0.01% of molybdenum oxide (Degussa Aerolyst 7751).

The support material of example catalyst E comprises 1% of yttrium oxide, 0.65% of iron oxide and 98.35% of silicon dioxide (Degussa Aerolyst 3045).

In the case of example catalyst F, a silicon dioxide containing 3.5% of lanthanum oxide and 6% of molybdenum oxide was used as support material and was impregnated with the active components palladium, gold and potassium acetate and, simultaneously therewith, 0.9% of iron oxide.

For example catalyst G, a support material comprising 1.5% of lanthanum oxide and 98.5% of titanium dioxide (Degussa Aerolyst 7751) was used and impregnated with the active components.

Comparative catalyst H comprises 1% of boron oxide and 99% of silicon dioxide as support material plus the active components palladium, gold and potassium acetate.

The following table reports the yield of vinyl acetate monomer as measure of the activity of the catalysts in gram of vinyl acetate monomer per hour and gram of palladium at 155°C, a pressure of 7 bar and a GHSV of 3000 h⁻¹:

| Catalyst | Yield [g/h·g] |
|---|---|
| A | 197.4 |
| B | 133.9 |
| C | 198.3 |
| D | 215.5 |
| E | 237.2 |
| F | 216.8 |
| G | 217.1 |
| H | 82.5 |

## Claims

1. Catalyst comprising palladium, gold and alkali metal acetate as catalytically active components on a support, **characterized in that** the catalyst is modified by means of titanium, iron, lanthanum, cerium, yttrium and/or molybdenum or oxides thereof.

2. Process for producing the catalyst according to Claim 1, **characterized in that** the support is impregnated with a salt of at least one doping component, calcined and subsequently impregnated with the catalytically active component or the support material is mixed with an oxide of the doping component, shaped and subsequently impregnated with the catalytically active components.

3. Process according to Claim 2, **characterized in that** the doping component is applied to the support simultaneously with the catalytically active component.

4. Use of the catalyst according to Claim 1 for preparing vinyl acetate monomer.
